# EUROPÄISCHE PATENTSCHRIFT

(11) **EP 1 556 434 B1**
(45) Veröffentlichungstag und Bekanntmachung des Hinweises auf die Patenterteilung: **17.10.2007**
(21) Anmeldenummer: 03788941.7
(22) Anmeldetag: 08.10.2003
(51) Int. Cl.: C08G 64/14, C08G 63/00, C07C 39/04, C07C 69/017, C07C 39/17, C07C 39/14, C07C 69/88

(54) **POLYCARBONATE, POLYESTERCARBONATE UND POLYESTER MIT LATERAL-STÄNDIGEN CYCLOALKYL-SUBSTITUIERTEN PHENOLEN**
POLYCARBONATES, POLYESTER CARBONATES AND POLYESTERS CONTAINING CYCLOALKYL-SUBSTITUTED PHENOLS IN A LATERAL POSITION
POLYCARBONATES, POLYESTERCARBONATES ET POLYESTERS CONTENANT DES PHENOLS SUBSTITUES PAR UN CYCLOALKYLE ET LATERAUX

(30) Priorität: 21.10.2002 DE 10248952
(43) Veröffentlichungstag der Anmeldung: 27.07.2005
(73) Patentinhaber: Bayer MaterialScience AG, 51368 Leverkusen (DE)
(72) Erfinder: BRUDER, Friedrich-Karl, 47802 Krefeld (DE); HEUER, Helmut-Werner, 47829 Krefeld (DE); MEYER, Alexander, 47800 Krefeld40237 Düsseldorf (DE); WEHRMANN, Rolf, 47800 Krefeld (DE)
(86) Internationale Anmeldenummer: PCT/EP2003/011108
(87) Internationale Veröffentlichungsnummer: WO 2004/037893

(56) Entgegenhaltungen:
- EP-A- 0 347 682
- EP-A- 0 794 209
- WO-A-98/22522
- GB-A- 1 291 411
- US-A- 4 624 802
- US-A- 4 699 971
- US-A- 5 043 403

## Beschreibung

Die vorliegende Erfindung betrifft die Verwendung von phenolischen Verbindungen mit o- und/oder m-Substituenten als Kettenabbrecher in Polycarbonaten, Polyestercarbonaten und Polyestern sowie Polycarbonate, Polyestercarbonate und Polyester mit von o- und/oder m-substituierten Phenolen abgeleiteten Endgruppen, Formkörper und Extrudate aus diesen Polymeren, Verfahren zur Herstellung der Polymere sowie Verfahren zur Herstellung der Formkörper und Extrudate.

Zur Herstellung von Polycarbonaten werden häufig monofunktionelle Verbindungen auf Phenolbasis wie z.B. Phenol, 4-Alkylphenole und 4-Cumylphenol als Kettenabbrecher eingesetzt (Kunststoff-Handbuch 3; L. Bottenbruch, Hanser, München 1992, S. 127; EP-A 0 353 594).

Polycarbonate mit von cycloalkylsubstituierten Phenolen abgeleiteten Endgruppen sind in allgemeiner Form bereits in US-A 46 99 971 und US-A 47 88 276 beschrieben. In US-A 46 99 971 und US-A 47 88 276 werden jedoch ausschließlich p-substituierte Cycloalkylphenole explizit offenbart und als besonders bevorzugt genannt.

Ester-funktionalisierte Endgruppen in Polycarbonat sind ebenfalls in allgemeiner Form bereits in CA 13 31 669 beschrieben. Explizit und vorzugsweise werden dort jedoch nur p- bzw. p,m-Carbonsäureester-substituierte Phenole beschrieben. JP-A 63 21 57 14 beschreibt Polycarbonate mit reaktiven Endgruppen wie OH- und COOH-Gruppen.

Speziell für das Schmelzeumesterungsverfahren werden in EP-A 09 76 772 Kettenabbrecher folgender Strukturen beschrieben: 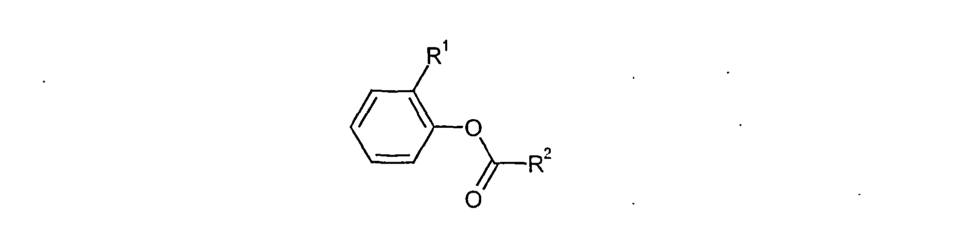 wobei
- R¹: für Chlor, methoxy- oder ethoxycarbonyl und
- R²: für einen Alkyl- oder Alkoxyrest oder für einen gegebenenfalls substituierten Aryl- oder Aryloxyrest steht.

Ebenenfalls für das Schmelzeumesterungsverfahren werden in EP-A 09 80 861 Salicylsäurederivate folgender Struktur beschrieben: 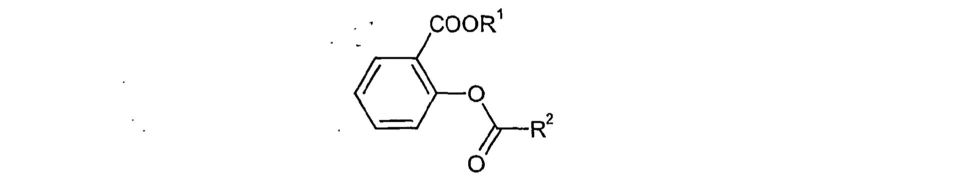 wobei
- R¹: für eine Methyl- oder Ethylgruppe und
- R²: für einen Alkyl-, Alkoxy-, Aryl- oder Aryloxyrest steht, welcher gegebenenfalls noch substituiert ist.

Lineare alkylsubstituierte und verzweigte alkylsubstituierte Endgruppen sind ebenfalls bekannt und z.B. in US-A 42 69 964 beschrieben. Polycarbonate mit Alkylaminoendgruppen sind in US-A 30 85 992 beschrieben. Polycarbonate mit Benzotriazol-substituierten Endgruppen sind aus JP 20 00 06 35 08 A2 bekannt.

Aus den US-A 3 166 606 und 3 173 891 ist beispielweise p-Phenylphenol als Kettenabbrecher für Polycarbonate bekannt. Aus US-A 43 30 663 sind Polyestercarbonate bekannt, in welchen 4-Butylbenzoesäurechlorid als Kettenabbrecher eingesetzt wird.

WO-A 00/50 488 beschreibt die Verwendung von Di-*tert*.-alkylphenol als Kettenabbrecher.

Aus der japanischen Offenlegungsschrift 57 13 31 49 sind Polycarbonate bekannt, die mit Phenylpropylphenol-, Alkylphenol- oder Naphtholresten als Endgruppen modifiziert sind.

In WO-A 01/05 866 werden Tritylphenol, Cumylphenol, Phenoxyphenol und Pentadecylphenol als Kettenabbrecher für Polycarbonat beschrieben.

Aus EP-A 10 48 684 und WO-A 99/36 458 sind Polycarbonate bekannt, welche beispielsweise mit 4-(1,1,3,3-Tetramethylbutyl)phenol und weiteren verzweigten Alkylphenolen modifiziert sind.

Gemäß DE-A 38 03 939 werden Kettenabbrecher der Formel 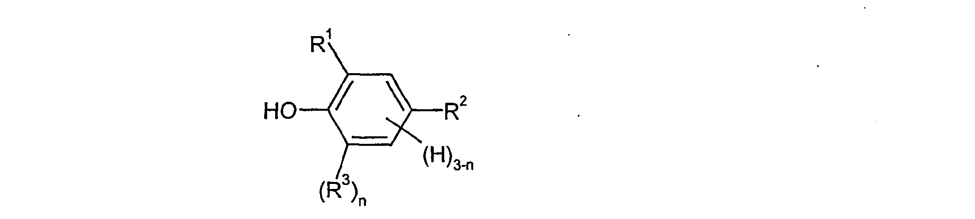 eingesetzt, worin
- R¹, R², R³: gleich oder verschieden sind und C₂ - C₁₂-Alkyl oder C₈-C₂₀-Aralkyl sind, wobei mindestens einer der Reste R¹ oder R² ein C₈ - C₂₀-Aralkyrest ist, und worin "n" einen Wert zwischen 0.5 und 1 hat.

Phenole mit cycloaliphatischen Resten werden nicht beschrieben. Vorteilhaft sollen 2,4 oder 2,4,6 substituierte Phenole sein. Es werden technische Gemische von Phenolen und keine Reinsubstanzen eingesetzt. Die Auswirkungen von Reinsubstanzen auf die Eigenschaften von Polycarbonat wird nicht beschrieben.

In EP-A 07 94 209 sind Polycarbonate mit Isooctylphenol- und Cumylphenolendgruppen beschrieben. JP-A 06 256 499 beschreibt Hydroxy-arylterminierte Polycarbonate.

Polycarbonate, Polyestercarbonate und Polyester mit den bekannten Endgruppen besitzen aber häufig den Nachteil einer relativ hohen Nullviskosität und/oder neigen bei thermischer Belastung zu Molekulargewichtsabbau bzw. Materialverfärbung.

Ausgehend vom Stand der Technik stellte sich somit die Aufgabe, Polycarbonate, Polyestercarbonate und Polyester mit Endgruppen, bzw. geeignete phenolische Verbindungen zur Erzeugung dieser Endgruppen, zu Verfügung zu stellen, welche den Nachteil einer hohen Nullviskosität vermeiden und damit über bessere Verarbeitungseigenschaften verfügen. Außerdem ist es wünschenswert, dass diese Endgruppen zu keinem Abbau unter thermischer Belastung wie z.B. im Extrusionsprozess oder im Spritzguss, aber auch im Herstellungsprozess bspw. nach dem Schmelzumesterungsverfahren, führen und somit z.B. auch im Schmelzumesterungsverfahren eingesetzt werden können.

Überraschenderweise wurde nun gefunden, dass diese Aufgabe durch den Einsatz von Kettenabbrechern mit o- und/oder m-ständigen Substituenten, insbesondere mit o- und/oder m-ständigen Cycloalkylsubstituenten, welche in p-Stellung nicht oder nur methylsubstituiert sind, gelöst wird. Diese Kettenabbrecher bzw. die daraus entstehenden Endgruppen im Polycarbonat beeinflussen die Nullviskosität überraschenderweise positiv, d.h. das entsprechende Polycarbonat zeigt bei vergleichbarer Molekulargewichtsverteilung eine niedrigere Nullviskosität und damit eine bessere Fließfähigkeit und ist daher besser verarbeitbar als Polycarbonate mit herkömmlichen.

Endgruppen. Polycarbonate, die an den von den Kettenabbrechern abgeleiteten Endgruppen o- und/oder m-substituiert sind, d.h. Polycarbonate, die z.B. o- und/oder m-substituierte Cycloalkylhydroxybenzoesäureester sowie o- und/oder m-substituierte Cycloalkylphenole als Endgruppen tragen, weisen überraschenderweise eine im Vergleich zu entsprechenden para-substituierten Verbindungen niedrigere Schmelzviskosität auf. Gegenüber herkömmlich eingesetzten Endgruppen wie z.B. p-tert.-Butylphenol oder Phenol wird ebenfalls eine Verbesserung der Schmelzviskosität erreicht.

Phenolische Kettenabbrecher für Polycarbonat, die mit Cycloalkylestern substituiert sind, sind bislang nicht bekannt.

Phenole mit Cycloalkylgruppen sind zwar bekannt (US-A 46 99 971 und US-A 47 88 276). In US-A 46 99 971 und US-A 47 88 276 werden allerdings explizit nur die p-Derivate als für den erfindungsgemäßen Zweck besonders geeignet offenbart. Im Gegensatz dazu sind jedoch überraschenderweise die erfindungsgemäßen o- und/oder m-substituierten Derivate den p-Derivaten des Standes der Technik deutlich überlegen

Gegenstand der vorliegenden Erfindung sind daher Polycarbonate, Polyestercarbonate und Polyester, die phenolische Endgruppen auf Basis von o- und/oder m-substituierten d.h. insbesondere o- und/oder m-cycloalkylsubstituierten Pheriolen, welche in p-Stellung nicht oder methylsubstituiert sind, bzw. auf Basis der entsprechenden o- oder m-verknüpften Ester enthalten, die Verwendung solcher Polycarbonate und spezielle, zur Verwendung in den erfindungsgemäßen Polycarbonaten geeignete phenolische Endgruppen.

Gegenstand der vorliegenden Erfmdung ist daher auch die Verwendung der phenolischen Verbindungen gemäß Formel (1) zur Herstellung endgruppenmodifizierter Polymere.

Die phenolischen Verbindungen der Formel (1) sind wie folgt definiert: 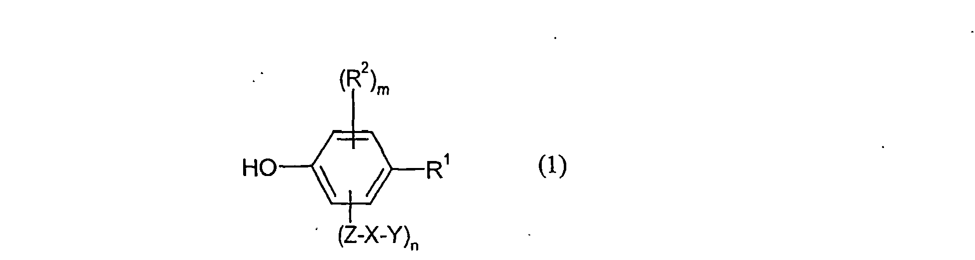 wobei
- R¹: entweder H oder ein CH₃-Rest, bevorzugt ein H ist;
- R²: für H, lineares oder verzweigtes C₁-C₁₈ Alkyl- oder Alkoxy-, Cl oder Br oder für einen gegebenenfalls substituierten Aryl- oder Aralkylrest, wobei die Substituenten bevorzugt Halogene, insbesondere F, Cl und Br sind, bevorzugt für H oder lineares oder verzweigtes C₁-C₁₂ Alkyl-, besonders bevorzugt für H oder C₁-C₈ Alkylrest und ganz besonders bevorzugt für H steht,
- Z: für einen Alkylspacer mit 1 bis 30 Kohlenstoffatomen oder für eine Einfachbindung, bevorzugt für einen Alkylspacer mit 1 bis 10 Kohlenstoffatomen oder eine Einfachbindung und ganz besonders bevorzugt für eine Einfachbindung steht,
- X: ein divalentes Radikal wie -O-, -CO-, -CH₂-, -COO- oder -OCO₂- steht,
- Y: für einen gegebenenfalls substituierten cycloaliphatischen Rest oder ein gegebenenfalls substituierten polycyclischen aliphatischen Rest wie Adamantyl- oder Norbornylrest oder einen gegebenenfalls substituierten aromatischen Rest, bevorzugt für einen gegebenenfalls substituierten cycloaliphatischen C₅-C₁₂ Rest oder für einen gegebenenfalls substituierten polycyclischen aliphatischen Rest wie Adamantyl- oder Norbornylrest oder für einen gegebenenfalls substituierten aromatischen Rest, besonders bevorzugt für einen gegebenenfalls substituierten cycloaliphatischen C₆-C₁₂ Rest oder gegebenenfalls substituierten Adamantyl- oder Norbornylrest oder für einen gegebenenfalls substituierten aromatischen Rest steht und ganz besonders bevorzugt für einen gegebenenfalls substituierten cycloaliphatischen C₆-C₁₂ Rest oder gegebenenfalls substituierten Adamantyl- oder Norbornylrest steht, wobei die Substituenten bevorzugt für Alkyl- oder Alkoxyrest und Halogene, insbesondere C1-C15 Alkyl- oder Alkoxyreste sowie F, Cl und Br stehen,
und
- n: die Werte 1,2,3,4 und m die Werte 0,1,2,3 annehmen kann, wobei n und m in Summe 4 ergeben müssen, wobei Verbindungen besonders bevorzugt sind in denen m = 3 und n = 1 ist.
oder
- X: für eine Einfachbindung steht und es sich im Falle, dass X für eine Einfachbindung steht bei den Verbindungen der Formel (1) um o-Cyclododecylphenol, o-Cyclooctylphenol, m-Cyclododecylphenol oder m-Cyclooctylphenol handelt.

Bevorzugt sind phenolische Verbindungen der Formel (1) die sich gemäß der Formeln (2), (3) und (4) näher bestimmen lassen:
Verbindungen der Formel (2) 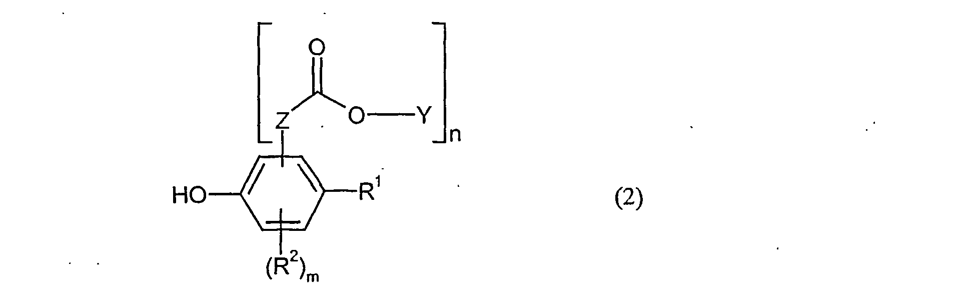 worin
- Z: für einen Alkylspacer mit 1 bis 30 Kohlenstoffatomen oder für eine Einfachbindung, bevorzugt für einen Alkylspacer mit 1 bis 10 Kohlenstoffatomen oder eine Einfachbindung und ganz besonders bevorzugt für eine Einfachbindung steht,
- R¹: für H oder einen Methylrest, bevorzugt ein H, steht,
- R²: für H, lineares oder verzweigtes C₁-C₁₈ Alkyl- oder Alkoxy-, Cl oder Br oder für einen gegebenenfalls substituierten Aryl- oder Aralkylrest, bevorzugt für H oder lineares oder verzweigtes C₁-C₁₂ Alkyl-, besonders bevorzugt für H oder C₁-C₈ Alkylrest und ganz besonders bevorzugt für H steht,
- Y: für einen gegebenenfalls substituierten cycloaliphatischen C₅-C₁₈ Rest oder für einen gegebenenfalls substituierten polycyclischen aliphatischen Rest wie Adamantyl- oder Norbornylrest oder für einen gegebenenfalls substituierten aromatischen Rest steht, bevorzugt für einen gegebenenfalls substituierten cycloaliphatischen C₅-C₁₂ Rest oder für einen gegebenenfalls substituierten polycyclischen aliphatischen Rest wie Adamantyl- oder Norbornylrest oder für einen gegebenenfalls substituierten aromatischen Rest, besonders bevorzugt für einen gegebenenfalls substituierten cycloaliphatischen C₆-C₁₂ Rest oder gegebenenfalls substituierten Adamantyl- oder Norbornylrest oder für einen gegebenenfalls substituierten aromatischen Rest steht und ganz besonders bevorzugt für einen gegebenenfalls substituierten cycloaliphatischen C₆-C₁₂ Rest oder gegebenenfalls substituierten Adamantyl- oder Norbornylrest steht,
und
- n: die Werte 1, 2, 3, 4 und m die Werte 0, 1, 2, 3 annehmen kann, wobei n und m in Summe 4 ergeben müssen, wobei Verbindungen besonders bevorzugt sind in denen m = 3 und n = 1 ist.
Verbindungen der Formel (3) 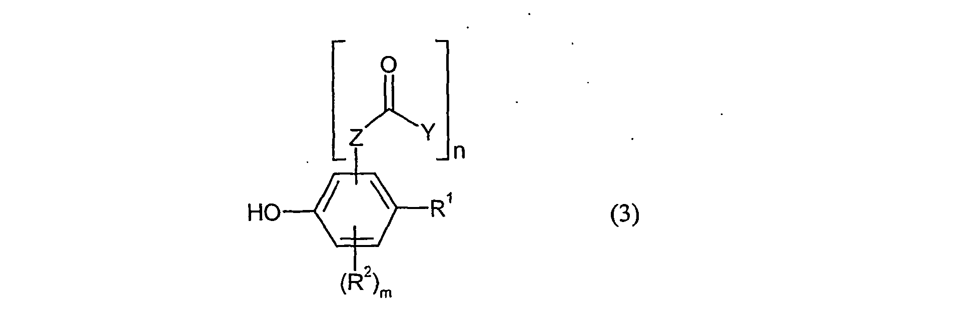 worin
- Z: für einen Alkylspacer mit 1 bis 30 Kohlenstoffatomen oder für eine Einfachbindung steht, bevorzugt für einen Alkylspacer mit 1 bis 10 Kohlenstoffatomen oder eine Einfachbindung und ganz besonders bevorzugt für eine Einfachbindung steht,
- R¹: für H oder einen Methylrest, bevorzugt ein H steht,
- R²: für H, lineares oder verzweigtes C₁-C₁₈ Alkyl- oder Alkoxy-, Cl oder Br oder für einen gegebenenfalls substituierten Aryl- oder Aralkylrest, bevorzugt für H oder lineares oder verzweigtes C₁-C₁₂ Alkyl-, besonders bevorzugt für H oder C₁-C₈ Alkylrest und ganz besonders bevorzugt für H steht,
- Y: für einen gegebenenfalls substituierten cycloaliphatischen C₅-C₁₈ Rest oder für einen gegebenenfalls substituierten polycyclischen aliphatischen Rest wie Adamantyl- oder Norbornylrest oder für einen gegebenenfalls substituierten aromatischen Rest steht, bevorzugt für einen gegebenenfalls substituierten cycloaliphatischen C₅-C₁₂ Rest oder für einen gegebenenfalls substituierten polycyclischen aliphatischen Rest wie Adamantyl- oder Norbornylrest oder für einen gegebenenfalls substituierten aromatischen Rest, besonders bevorzugt für einen gegebenenfalls substituierten cycloaliphatischen C₆-C₁₂ Rest oder gegebenenfalls substituierten Adamantyl- oder Norbornylrest oder für einen gegebenenfalls substituierten aromatischen Rest steht und ganz besonders bevorzugt für einen gegebenenfalls substituierten cycloaliphatischen C₆-C₁₂ Rest oder gegebenenfalls substituierten Adamantyl- oder Norbornylrest steht,
und
- n: die Werte 1, 2, 3, 4 und m die Werte 0, 1, 2, 3 annehmen kann, wobei n und m in Summe 4 ergeben müssen, wobei Verbindungen besonders bevorzugt sind in denen m = 3 und n = 1 ist.
Verbindungen der Formel (4) 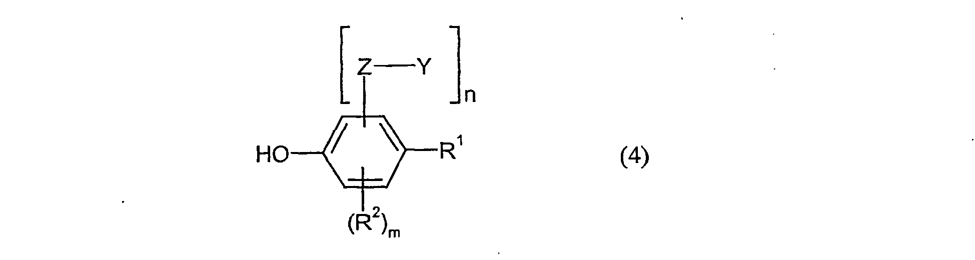 worin
- Z, Y, R¹ und R²: die oben genannte Bedeutung besitzen und
- n: die Werte 1, 2, 3, 4 und m die Werte 0, 1, 2, 3 annehmen kann, wobei n und m in Summe 4 ergeben müssen, wobei Verbindungen besonders bevorzugt sind in denen m = 3 und n = 1 ist.

Ganz besonders bevorzugt sind jeweils unabhängig voneinander die phenolischen Verbindungen, welche den Formeln (2a), (2b), (3a), (3b), (4a) und (4b) entsprechen: 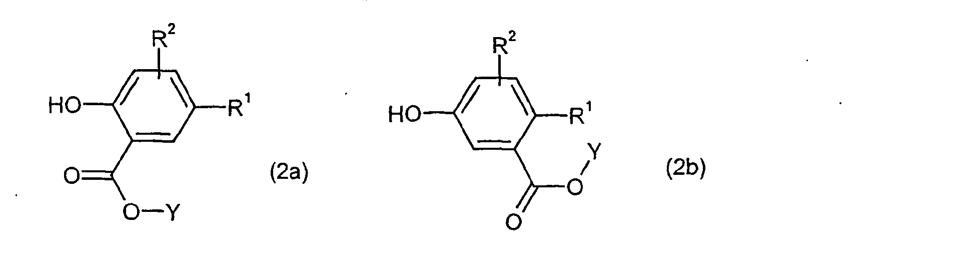 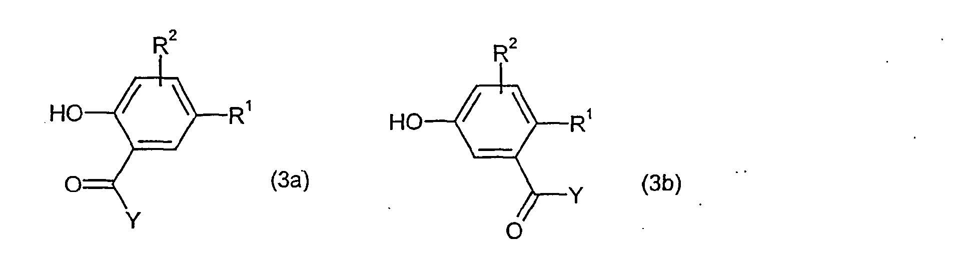 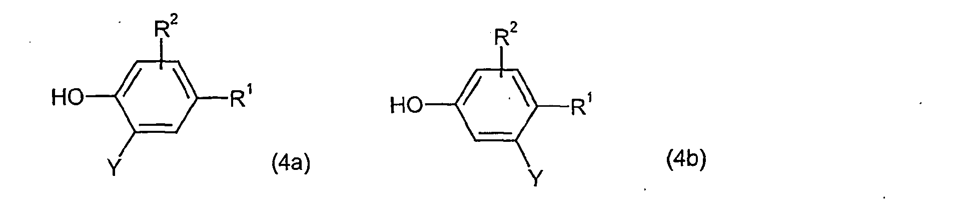 wobei in (2a), (2b), (3a), (3b), (4a) und (4b) die Reste R¹, R² und Y die oben genannten Bedeutungen haben.

Geeignete, von den phenolischen Verbindungen der Formeln (1)-(4) abgeleitete Endgruppen zur Modifikation von Polycarbonaten, Polyestercarbonaten und Polyestern sind durch Formel (5) dargestellt: 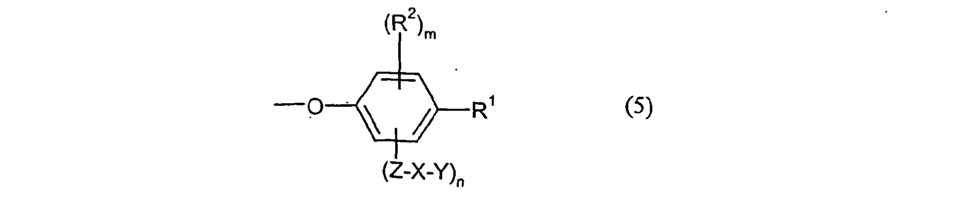 wobei die Reste und Substitutionsmuster wie oben für Formel (1) definiert, gelten.

Besonders geeignet sind die Endgruppen der Formel (5a) bis (5f) 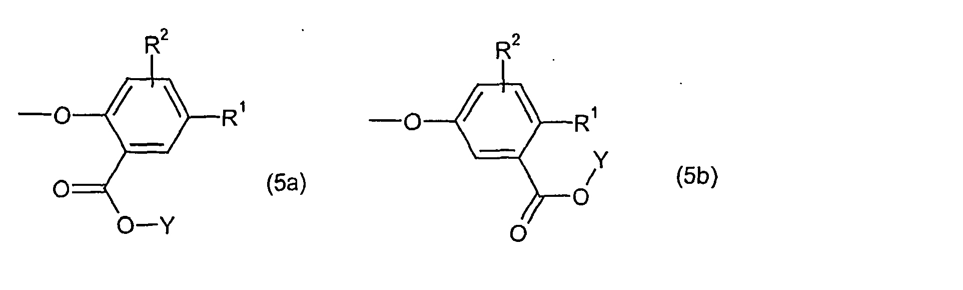 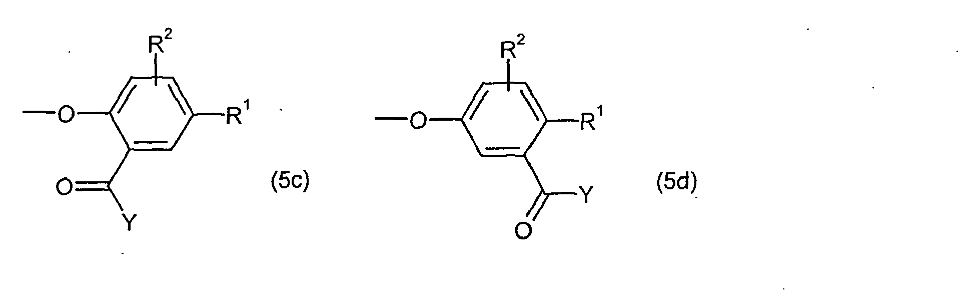 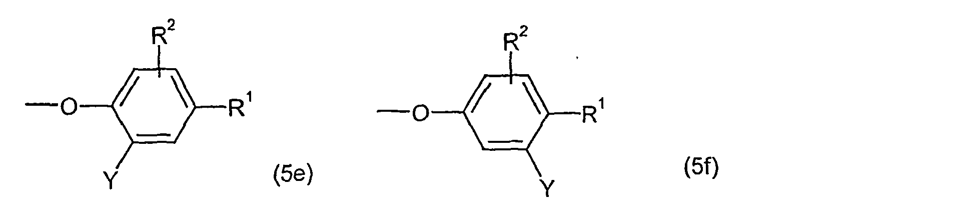 worin die Reste Y, R¹ und R² die oben genannten Bedeutungen haben und es sich im Falle der Formeln (5e) und (5f) um die aus o-Cyclododecylphenol, o-Cyclooctylphenol, m-Cyclododecylphenol oder m-Cyclooctylphenol resultierenden Endgruppen handelt.

Unabhängig voneinander ganz besonders geeignet sind die den phenolischen Verbindungen der Formeln (2a), (2b), (3a), (3b), (4a) und (4b) entsprechenden Endgruppen.

Bevorzugt, besonders bevorzugt, ganz besonders bevorzugt oder insbesondere bevorzugt etc. sind Verbindungen, welche die unter bevorzugt, besonders bevorzugt, ganz besonders bevorzugt oder insbesondere bevorzugt etc. genannten Substituenten tragen.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend.

Gegenstand der vorliegenden Erfindung sind somit auch thermoplastische Polycarbonate, thermoplastische Polyestercarbonate und thermoplastische Polyester mit den phenolischen Verbindungen der Formeln (1), (2), (3) und (4) entsprechenden Endgruppen.

Beispiele für phenolische Verbindungen der Formel (1) sind o-Cyclododecylphenol, o-Cyclooctylphenol, m-Cyclododecylphenol, m-Cyclooctylphenol, 3-Hydroxybenzophenon, 2-Hydroxybenzophenon, 3-Hydroxybenzoesäurephenylester, 2-Hydroxybenzoesäurephenylester, 3-Hydroxybenzoesäure-(4-tert.-butylphenyl)ester, 2-Hydroxybenzoesäure-(4-tert.-butylphenyl)-ester, 3-Hydroxybenzoesäure-(4-methylphenyl)ester, 2-Hydroxybenzoesäure-(4-methylphenyl)ester, 3-Hydroxybenzoesäurecyclohexylester, 2-Hydroxybenzoesäurecyclohexylester 3-Hydroxybenzoesäurecyclooctylester, 2-Hydroxybenzoesäurecyclooctylester 3-Hydroxybenzoesäurecyclododecylester und 2-Hydroxybenzoesäurecyclododecylester.

Cycloalkylphenole sind generell in der Literatur bekannt (bspw. FR-A 15 80 640, US-A 46 99 971 und US-A 47 88 276). Ebenso sind Hydroxybenzophenone (bspw. EP-A 32 27 5) und Hydroxybenzoesäurealkylester (DE-A 34 00 342 ) bekannt. Hydroxybenzoesäurecycloalkylester sind ebenfalls bekannt und z.B. in DE-A 34 00 342, JP 60145882 A2 und JP 06001913 A2 beschrieben.

Die erfindungsgemäßen Verbindungen sind prinzipiell nach bekannten Methoden der organischen Chemie herstellbar.

Beispielweise lassen sich o-Cycloalhylester-substituierte Phenole der Formel (5a) durch Umsetzung von Salicylsäureestern wie z.B. Salicylsäuremethylester mit entsprechenden Alkoholen unter Zusatz von Base wie z.B. Kaliumcarbonat oder Natriummethylat oder unter Zusatz von Umesterungskatalysatoren wie z.B. Titantetraisopropylat darstellen. Entsprechende m-substituierte Verbindungen der Formel (5b) erfordern den Einsatz des entsprechenden m-Hydroxybenzoesäuremethylesters. Bei diesen Reaktionen empfiehlt es sich, den leichtsiedenden Alkohol während der Reaktion abzudestillieren (s.a. DE-A 34 00 342).

Verbindungen der Formel (5c) bzw. (5d) lassen sich durch Friedel-Crafts-Acylierung wie z.B. durch Umsetzung von gegebenenfalls substituierten Anisolderivaten mit Säurechloriden unter Zusatz von Metallsalzen wie z.B. FeCl₃ oder AlCl₃ erhalten. In einem zweiten Schritt muss die phenolische OH Gruppe durch Spaltung des Methylethers freigesetzt werden. Dies kann z.B. mit BBr₃ oder HBr erfolgen (Ether-Spaltungen sind z.B. beschrieben von A. Kamai, N. L. Gayatri, Tetrahedron Lett. 1996, 37, 3359; Friedel-Crafts-Reaktionen sind z.B. beschrieben von H. Heaney, Comprehensive Organic Chemistry; Ed.: B. M. Trost; Pergamon Press Oxford 1991, Vol. 2, S. 753).

Verbindungen der Formel (5e) bzw. (5f) lassen sich prinzipiell durch Reaktion von Phenolen mit Cycloalkenen wie z.B. Cyclohexen oder Cycloocten bei Temperaturen zwischen 250 und 350°C gegebenenfalls unter Zusatz von Säuren wie z.B. Schwefelsäure oder BF₃ herstellen (diese Reaktionen sind z.B. von W. Jones, J. Org. Chem. 1953, 4156 oder von Kolka et aL, J. Org. Chem. 1957, 22, 642 beschrieben).

Außer den phenolischen Verbindungen der Formel (1), (2), (3) und (4) können noch andere Phenole in Mengen bis zu 50 mol-% bezogen auf die jeweilige Gesamtmenge an Kettenabbrecher zur Herstellung der Polycarbonate, Polyestercarbonate und Polyester mitverwendet werden.

Gegenstand der vorliegenden Erfindung ist somit auch die Verwendung der phenolischen Verbindungen der Formel (1) gegebenenfalls in Kombination mit anderen Phenolen als Kettenabbrecher zur Herstellung von aromatischen Polycarbonate, aromatischen Polyestercarbonaten und aromatischen Polyestern, wobei die anderen Phenole in Mengen bis zu 50 mol-% vorzugsweise bis 25 mol-% bezogen auf die jeweilige Gesamtmolmenge an Kettenabbrecher eingesetzt werden.

Gegenstand der vorliegenden Erfindung sind somit auch thermoplastische Polycarbonate, thermoplastische Polyestercarbonate und thermoplastische Polyester enthaltend von den phenolischen Verbindungen der Formeln (1), (2), (3) und (4) abgeleitete Endgruppen, beispielhaft aber nicht einschränkend durch die Polymere der Formel (6) dargestellt, 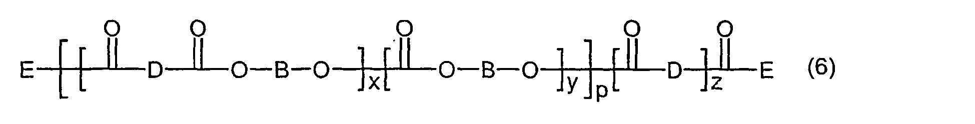 worin 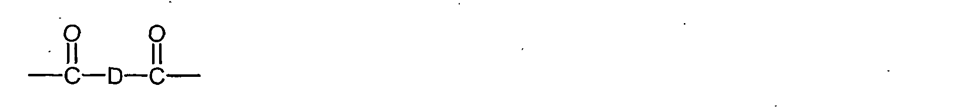 der Rest einer aromatischen Dicarbonsäure ist, -O-B-O- ein Bisphenolatrest ist, "p" eine ganze Zahl zwischen 25 und 700 ist, "x" und "y" Bruchzahlen aus der Reihe 0/p, 1/p, 2/p bis p/p sind, wobei x + y = 1 ist und "z" = Null oder 1 ist und mindestens 50 mol-% der Reste E in (6) den Phenolatresten entsprechend den phenolischen Verbindungen der Formel (1), (2), (3) und (4) entsprechen und maximal 50 mol-% der Reste E in (6) einem anderen Phenolatrest als dem entsprechend den phenolischen Verbindungen der Formel (1), (2), (3) oder (4) entsprechen.

Gemäß DE-A 2 119 799 erfolgt die Herstellung von Polycarbonaten unter Beteiligung phenolischer Endgruppen, nach dem Phasengrenzflächenverfahren wie auch dem Verfahren in homogener Phase.

Zur Herstellung von Polycarbonaten nach dem Phasengrenzflächenverfahren sei beispielhaft auf H. Schnell, Chemistry and Physics of Polycarbonates, Polymer Reviews, Vol. 9, Interscience Publishers, New York 1964 S. 33 ff. und auf Polymer Reviews, Vol. 10, "Condensation Polymers by Interfacial and Solution Methods", Paul W. Morgan, Interscience Publishers, New York 1965, Kap. VIII, S. 325 verwiesen.

Daneben ist die Herstellung der erfindungsgemäßen Polycarbonate auch aus Diarylcarbonaten und Diphenolen nach dem bekannten Polycarbonatverfahren in der Schmelze, dem sogenannten Schmelzumesterungsverfahren, möglich, das z.B. in WO-A 01/05866 und WO-A 01/05867 beschrieben ist. Daneben werden Umesterungsverfahren (Acetatverfahren und Phenylesterverfahren) beispielsweise in den US-A 34 94 885, 43 86 186, 46 61 580, 46 80 371 und 46 80 372, in den EP-A 26 120, 26 121, 26 684, 28 030, 39 845, 39 845, 91 602, 97 970, 79 075, 14 68 87, 15 61 03, 23 49 13 und 24 03 01 sowie in den DE-A 14 95 626 und 22 32 977 beschrieben. Auch in diesen Verfahren ist die erfindungsgemäße Verwendung von phenolischen Verbindungen mit o- und/oder m-Substituenten als Kettenabbrecher möglich.

Diarylcarbonate sind solche Kohlensäurediester der Formel (7) 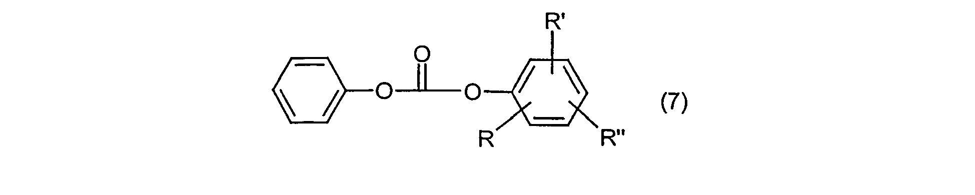 und Formel (8), 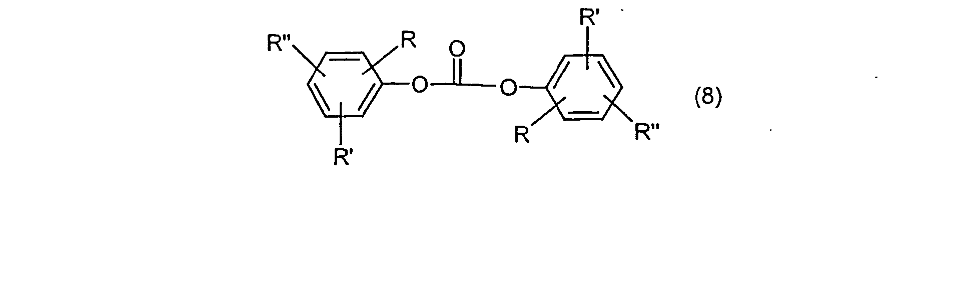 wobei R, R' und R" unabhängig voneinander H, gegebenenfalls verzweigte C₁-C₃₄-Alkyl/Cycloalkyl, C₇-C₃₄-Alkaryl oder C₆-C₃₄-Aryl bzw. C₆-C₃₄-Aryloxy darstellen können, beispielsweise

Diphenylcarbonat, Butylphenyl-phenylcarbonat, Di-butylphenylcarbonat, Isobutylphenyl-phenylcarbonat, Di-isobutylphenylcarbonat, tert-Butylphenyl-phenylcarbonat, Di-tert-butylphenylcarbonat, n-Pentylphenyl-phenylcarbonat, Di-(n-pentylphenyl)carbonat, n-Hexylphenyl-phenylcarbonat, Di-(n-hexylphenyl)carbonat, Cyclohexylphenyl-phenylcarbonat, Di-cyclohexylphenylcarbonat, Phenylphenol-phenylcarbonat, Di-phenylphenolcarbonat, Isooctylphenyl-phenylcarbonat, Di-isooctylphenylcarbonat, n-Nonylphenyl-phenylcarbonat, Di-(n-nonylphenyl)carbonat, Cumylphenyl-phenylcarbonat, Di-cumylphenylcarbonat, Naphthylphenyl-phenylcarbonat, Di-naphthylphenylcarbonat, Di-tert-butylphenyl-phenylcarbonat, Di-(di-tert-butylphenyl)carbonat, Dicumylphenyl-phenylcarbonat, Di-(dicumylphenyl)-carbonat, 4-Phenoxyphenyl-phenylcarbonat, Di-(4-phenoxyphenyl)carbonat, 3-Pentadecylphenyl-phenylcarbonat, Di-(3-pentadecylphenyl)carbonat, Tritylphenylphenylcarbonat, Di-tritylphenylcarbonat,
bevorzugt
Diphenylcarbonat, tert-Butylphenyl-phenylcarbonat, Di-tert-butylphenylcarbonat, Phenylphenol-phenylcarbonat, Di-phenylphenolcarbonat, Cumylphenyl-phenylcarbonat, Di-cumylphenylcarbonat,
besonders bevorzugt Diphenylcarbonat.

Diphenole für die erfindungsgemäßen Polycarbonate können beispielsweise Hydrochinon, Resorcin, Dihydroxybiphenyle, Bis-(hydroxyphenyl)-alkane, Bis-(hydroxyphenyl)-cycloalkane, Bis-(hydroxyphenyl)-sulfide, Bis-(hydroxyphenyl)-ether, Bis-(hydroxyphenyl)-ketone, Bis-(hydroxyphenyl)-sulfone, Bis-(hydroxyphenyl)-sulfoxide, α,α'-Bis-(hydroxyphenyl)-diisopropylbenzole, sowie deren kemalkylierte und kernhalogenierte Verbindungen sein und auch α,ω- Bis-(hydroxyphenyl)-polysiloxane.

Bevorzugte Diphenole sind beispielsweise 4,4'-Dihydroxybiphenyl (DOD), 2,2-Bis-(4-hydroxyphenyl)-propan (Bisphenol A), 1,1-Bis-(4-hydroxyphenyl)-3,3,5-trimethylcyclohexan (Bisphenol TMC), 1,1-Bis-(4-hydroxyphenyl)-cyclohexan, 2,4-Bis-(4-hydroxyphenyl)-2-methylbutan, 1,1-Bis-(4-hydroxyphenyl)-1-phenylethan, 1,1-Bis-(4-hydroxyphenyl)-*p*-diisopropylbenzol, 1,3-Bis[2-(4-hydroxyphenyl)-2-propyl]-benzol (Bisphenol M), 2,2-Bis-(3-methyl-4-hydroxyphenyl)-propan, 2,2-Bis-(3-chlor-4-hydroxyphenyl)-propan, Bis-(3,5-dimethyl-4-hydroxyphenyl)-methan, 2,2-Bis-(3,5-dimethyl-4-hydroxyphenyl)-propan, Bis-(3,5-dimethyl-4-hydroxyphenyl)-sulfon, 2,4-Bis-(3,5-dimethyl-4-hydroxyphenyl)-2-methylbutan, 2,2-Bis-(3,5-dichlor-4-hydroxyphenyl)-propan und 2,2-Bis-(3,5-dibrom-4-hydroxyphenyl)-propan.

Besonders bevorzugte Diphenole sind beispielsweise 2,2-Bis-(4-hydroxyphenyl)-propan (Bisphenol A), 1,3-Bis[2-(4-hydroxyphenyl)-2-propyl]-benzol (Bisphenol M), 2,2-Bis-(3,5-dimethyl-4-hydroxyphenyl)-propan, 1,1-Bis-(4-hydroxyphenyl)-1-phenylethan, 2,2-Bis-(3,5-dichlor-4-hydroxyphenyl)-propan, 2,2-Bis-(3,5-dibrom-4-hydroxyphenyl)-propan, 1,1-Bis-(4-hydroxyphenyl)-cyclohexan und 1,1-Bis-(4-hydroxyphenyl)-3,3,5-trimethylcyclohexan (Bisphenol TMC).

Ganz besonders bevorzugt sind 2,2-Bis-(4-hydroxyphenyl)-propan (Bisphenol A), 1,3-Bis[2-(4-hydroxyphenyl)-2-propyl]-benzol (Bisphenol M) und 1,1-Bis-(4-hydroxyphenyl)-3,3,5-trimethylcyclohexan (Bisphenol TMC).

Die Diphenole können sowohl allein als auch im Gemisch miteinander verwendet werden; es sind sowohl Homopolycarbonate als auch Copolycarbonate einbezogen. Die Diphenole sind literaturbekannt oder nach literaturbekannten Verfahren herstellbar (siehe z.B. H. J. Buysch et al., Ullmann's Encyclopedia of Industrial Chemistry, VCH, New York 1991, 5. Ed., Vol. 19, p. 348).

Es können auch geringe Mengen, vorzugsweise Mengen zwischen 0.05 und 2.0 mol-%, bezogen auf die Mole eingesetzter Diphenole, an tri- oder multifunktionellen Verbindungen, insbesondere solchen mit drei oder mehr als drei phenolischen Hydroxygruppen als sogenannte Verzweiger, mitverwandt werden. Dadurch ergeben sich selbstverständlich Abweichungen von der idealisierten und nur beispielhaft angeführten Formel (6), da es dann zu verzweigenden Strukturen in Abweichung von den angegebenen Strukturen D und B kommt.

Einige der verwendbaren Verbindungen mit drei oder mehr als drei phenolischen Hydroxygruppen sind beispielsweise Phloroglucin, 4,6-Dimethyl-2,4,6-tri-(4-hydroxyphenyl)-hept-2-en, 4,6-Dimethyl-2,4,6-tri-(4-hydroxyphenyl)-heptan, 1,3,5-Tri-(4-hydroxyphenyl)-benzol, 1,1,1-Tri-(4-hydroxyphenyl)-ethan, Tri-(4-hydroxyphenyl)-phenylmethan, 2,2-Bis-[4,4-bis-(4-hydroxyphenyl)-cyclohexyl]-propan, 2,4-Bis-(4-hydroxyphenyl-isopropyl)-phenol, 2,6-Bis-(2-hydroxy-5'-methylbenzyl)-4-methylphenol, 2-(4-Hydroxyphenyl)-2-(3,4-dihydroxyphenyl)-propan, Hexa-[4-(4-hydroxyphenyl-isopropyl)-phenyl]-orthoterephthalsäureester, Tetra-[4-(4-hydroxyphenyl-isopropyl)-phenoxy]-methan, Tetra-(4-hydroxyphenyl)-methan und 1,4-Bis-(4',4''-dihydroxytriphenyl)-methylbenzol.

Weitere mögliche Verzweiger sind 2,4-Dihydroxybenzoesäure, Trimesinsäure, Cyanurchlorid und 3,3-Bis-(3-methyl-4-hydroxyphenyl)-2-oxo-2,3-dihydroindol.

Die gegebenenfalls mitzuverwendenden 0.05 bis 2 mol-%, bezogen auf eingesetzte Diphenole, an Verzweigern können entweder mit den Diphenolen selbst und den erfindungsgemäßen Molekulargewichtsreglern in der wässrigen alkalischen Phase vorgelegt werden, oder in einem organischen Lösungsmittel gelöst vor der Phosgenierung zugegeben werden.

Die aromatischen Polycarbonate der vorliegenden Erfindung besitzen Gewichtsmittelmolekulargewichte M_{w} (ermittelt durch Gelpermeationschromatographie und Eichung mit Polystyrolstandard) zwischen 5000 und 200000, vorzugsweise zwischen 10000 und 80000 und besonders bevorzugt zwischen 15000 und 40000.

Die relativen Lösungsviskositäten liegen entsprechend zwischen 1.10 und 1.60 gemessen in Methylenchlorid (0.5 g Polycarbonat in 100 ml Methylenchlorid bei 23°C).

Erfindungsgemäße Polyestercarbonate sind solche, die aus mindestens einem Diphenol, aus mindestens einer aromatischen Dicarbonsäure und aus Kohlensäure aufgebaut sind.

Geeignete aromatische Dicarbonsäuren sind beispielsweise Orthophthalsäure, Terephthalsäure, Isophthalsäure, *tert*.-Butylisophthalsäure, 3,3'-Diphenyldicarbonsäure, 4,4'-Diphenyletherdicarbonsäure, 4,4'-Diphenylsulfondicarbonsäure, 3,4'-Benzophenondicarbonsäure, 2,2-Bis-(4-carboxyphenyl)-propan, Trimethyl-3-phenylindan-4,5-dicarbonsäure.

Von den aromatischen Dicarbonsäuren werden besonders bevorzugt die Terephthalsäure und/oder Isophthalsäure eingesetzt.

Geeignete Diphenole sind die vorstehend für die Polycarbonatherstellung genannten. Die Kohlensäure kann entweder via Phosgen oder via Diphenylcarbonat in die Polyestercarbonate eingebaut werden, je nach Wahl des Herstellungsverfahrens, also je nachdem, ob Phasengrenzflächenpolykondensation oder Schmelzumesterung zur Polyestercarbonatherstellung verwendet wird.

Entsprechendes gilt für die aromatischen Dicarbonsäuren; sie werden entweder als aromatische Dicarbonsäuredichloride im Zweiphasengrenzflächenverfahren oder als Dicarbonsäurediester im Schmelzumesterungsverfahren eingesetzt.

Die Herstellung der erfindungsgemäßen Polyestercarbonate erfolgt nach bekannten Herstellungsverfahren, also wie bereits erwähnt beispielsweise nach dem Phasengrenzflächenverfahren oder nach dem Schmelzumesterungsverfahren.

Die erfindungsgemäßen Polyestercarbonate können sowohl linear als auch in bekannter Weise verzweigt sein. Die aromatischen Polyestercarbonate der vorliegenden Erfindung haben mittlere Gewichtsmittelmolekulargewichte M_{w} (ermittelt durch Gelpermeationschromatographie mit Polystyroleichung) vorzugsweise zwischen 10000 und 250 000.

Das molare Verhältnis von Carbonateinheiten zu aromatischen Dicarboxylateinheiten in den erfindungsgemäßen Polyestercarbonaten liegt vorzugsweise bei 95:5 bis 5:95, besonders bevorzugt bei 90:10 bis 10:90, insbesondere bevorzugt bei 80:20 bis 20:80 und ganz besonders bevorzugt bei 60:40 bis 40:60 vor.

"z" kann im Falle der erfindungsgemäßen Polyester (6) sowohl 0 als auch 1 sein.

Erfindungsgemäße aromatische Polyester sind solche aus mindestens einem Diphenol und mindestens einer aromatischen Dicarbonsäure.

Geeignete Diphenole und Dicarbonsäuren sind die vorstehend für die Polyestercarbonatherstellung genannten.

Die erfindungsgemäßen aromatischen Polyester werden nach bekannten Herstellungsverfahren (siehe z.B. Kunststoff-Handbuch, Bd. VIII, S. 695 ff, Carl-Hanser-Verlag, München, 1973) hergestellt.

Die erfindungsgemäßen aromatischen Polyester können sowohl linear als auch in bekannter Weise verzweigt sein.

Die erfindungsgemäßen aromatischen Polyester besitzen mittlere Gewichtsmittelmolekulargewichte M_{w} (ermittelt nach der Lichtstreuungsmethode), vorzugsweise zwischen 25 000 und 70 000; dies entspricht Polymerisationsgraden "p" in Formel (6) von etwa 80 bis 270, wobei "x" = 1, "y" = 0 und z = 1 sind.

Die einzusetzende Menge an erfindungsgemäßen Monophenolen der Formel (1), (2) oder (3) zur Herstellung der erfindungsgemäßen Polycarbonate, Polyestercarbonate oder Polyester liegt zwischen 0.5 mol-% und 8 mol-%, vorzugsweise zwischen 2 mol-% und 6 mol-% bezogen auf die jeweils eingesetzten Diphenole.

Als weitere Kettenabbrecher sind die üblichen Monophenole wie beispielsweise Phenol, 4-Alkylphenole und 4-Cumylphenol geeignet.

Gegenstand der vorliegenden Erfindung ist somit auch ein Verfahren zur Herstellung der erfindungsgemäßen Polycarbonate, Polyestercarbonate oder Polyester aus Diphenolen, Monophenolen, Kohlensäurederivaten und/oder Dicarbonsäurederivaten nach an sich bekannten Verfahrensbedingungen, das dadurch gekennzeichnet ist, dass man als Kettenabbrecher Monophenole der Formel (1), (2), (3) oder (4) in Mengen von 0.5 mol-% bis 8 mol-%, vorzugsweise von 2 mol-% bis 6 mol-% , bezogen jeweils auf mole Diphenole, einsetzt, wobei davon bis zu 50 mol-%, vorzugsweise bis zu 25 mol-%, bezogen jeweils auf die Gesamtmenge an Kettenabbrecher, durch andere Monophenole ersetzt werden können.

Im Falle des Phasengrenzflächenpolykondensationsverfahrens können die Kettenabbrecher der Formel (1), (2), (3) oder (4) vor, während oder nach der Phosgenierung in Lösung zugesetzt werden. Die für das Lösen der Kettenabbrecher der Formel (1), (2), (3) oder (4) geeigneten Lösungsmittel sind beispielsweise Methylenchlorid, Chlorbenzol oder Acetonitril sowie Mischungen dieser Lösungsmittel.

Im Falle des Schmelzeumesterungsverfahren besteht nach dem erfindungsgemäßen Verfahren die Möglichkeit, die Kettenabbrecher der Formel (1), (2), (3) oder (4) zu jedem Zeitpunkt der Reaktion zuzugeben; dabei kann die Zugabe in mehrere Portionen aufgeteilt werden.

Gegenstand der Erfindung sind auch die nach dem erfindungsgemäßen Verfahren erhältlichen Polycarbonate, Polyestercarbonate und Polyester.

Diphenole zur Herstellung der erfindungsgemäßen Polycarbonate, Polyestercarbonate und Polyester können auch Polymerisate oder Kondensate mit phenolischen Endgruppen sein, so dass erfindungsgemäß auch Polycarbonate bzw. Polyestercarbonate bzw. Polyester mit Blockstrukturen einbezogen sind.

Die erfindungsgemäßen Polycarbonate, Polyestercarbonate und Polyester können in bekannter Weise aufgearbeitet und zu beliebigen Formkörpern verarbeitet werden, beispielsweise durch Extrusion oder Spritzguss.

Den erfindungsgemäßen Polycarbonaten, Polyestercarbonaten und Polyestern können noch andere aromatische Polycarbonate und/oder andere aromatische Polyestercarbonate und/oder andere aromatische Polyester in bekannter Weise zugemischt werden.

Den erfindungsgemäßen Polycarbonaten, Polyestercarbonaten und Polyestern können noch die für diese Thermoplasten üblichen Additive wie Füllstoffe, UV-Stabilisatoren, Thermostabilisatoren, Antistatika und Pigmente in den üblichen Mengen zugesetzt werden; gegebenenfalls können das Entformungsverhalten, das Fließverhalten, und/oder die Flammwidrigkeit noch durch Zusatz externer Entformungsmittel, Fließmittel, und/oder Flammschutzmittel verbessert werden (z.B. Alkyl- und Arylphosphite, -phosphate, -phosphane, -niedermolekulare Carbonsäureester, Halogenverbindungen, Salze, Kreide, Quarzmehl, Glas- und Kohlenstofffasern, Pigmente und deren Kombination. Solche Verbindungen werden z.B. in WO 99/55772, S. 15 - 25, und in "Plastics Additives", R. Gächter und H. Müller, Hanser Publishers 1983, beschrieben).

Die erfindungsgemäßen Polycarbonate, Polyestercarbonate und Polyester, gegebenenfalls in Abmischung mit anderen Thermoplasten und/oder üblichen Additiven können zu beliebigen Formkörpern/Extrudaten verarbeitet überall dort eingesetzt werden, wo bereits bekannte Polycarbonate, Polyestercarbonate und Polyester eingesetzt werden. Aufgrund ihres Eigenschaftsprofils eignen sie sich insbesondere als Substratmaterialien für optische Datenspeicher wie z.B. CD, CD-R, DVD, oder DVD-R, sind aber auch beispielsweise als Folien im Elektrosektor als Formteile im Fahrzeugbau und als Platten für Abdeckungen im Sicherheitsbereich einsetzbar. Weitere mögliche Anwendungen der erfindungsgemäßen Polycarbonate sind:
1. Sicherheitsscheiben, die bekanntlich in vielen Bereichen von Gebäuden, Fahrzeugen und Flugzeugen erforderlich sind, sowie als Schilde von Helmen.
2. Herstellung von Folien, insbesondere Skifolien.
3. Herstellung von Blaskörpern (siehe beispielsweise US-Patent 2 964 794), beispielsweise 1 bis 5 Gallon Wasserflaschen.
4. Herstellung von lichtdurchlässigen Platten, insbesondere von Hohlkammerplatten, beispielsweise zum Abdecken von Gebäuden wie Bahnhöfen, Gewächshäusern und Beleuchtungsanlagen.
5. Herstellung optischer Datenspeicher.
6. Zur Herstellung von Ampelgehäusen oder Verkehrsschildern.
7. Zur Herstellung von Schaumstoffen (siehe beispielsweise DE-B 1 031 507).
8. Zur Herstellung von Fäden und Drähten (siehe beispielsweise DE-B 1 137 167 und DE-A 1 785 137).
9. Als transluzente Kunststoffe mit einem Gehalt an Glasfasern für lichttechnische Zwecke (siehe beispielsweise DE-A 1 554 020).
10. Als transluzente Kunststoffe mit einem Gehalt an Bariumsulfat, Titandioxid und oder Zirkoniumoxid bzw. organischen polymeren Acrylatkautschuken (EP-A 634 445, EP-A 269324) zur Herstellung von lichtdurchlässigen und lichtstreuenden Formteilen.
11. Zur Herstellung von Präzisionsspritzgussteilchen, wie beispielsweise Linsenhalterungen. Hierzu verwendet man Polycarbonate mit einem Gehalt an Glasfasern, die gegebenenfalls zusätzlich etwa 1 - 10 Gew.-% MoS₂, bezogen auf Gesamtgewicht, enthalten.
12. Zur Herstellung optischer Geräteteile, insbesondere Linsen für Foto- und Filmkameras (siehe beispielsweise DE-A 2 701 173).
13. Als Lichtübertragungsträger, insbesondere als Lichtleiterkabel (siehe beispielsweise EP-A 0 089 801).
14. Als Elektroisolierstoffe für elektrische Leiter und für Steckergehäuse sowie Steckverbinder.
15. Herstellung von Mobiltelefongehäusen mit verbesserter Beständigkeit gegenüber Parfüm, Rasierwasser und Hautschweiß.
16. Network interface devices
17. Als Trägermaterial für organische Fotoleiter.
18. Zur Herstellung von Leuchten, z.B. Scheinwerferlampen, als sogenannte "headlamps", Streulichtscheiben oder innere Linsen.
19. Für medizinische Anwendungen, z.B. Oxygenatoren, Dialysatoren.
20. Für Lebensmittelanwendungen, wie z.B. Flaschen, Geschirr und Schokoladenformen.
21. Für Anwendungen im Automobilbereich, wo Kontakt zu Kraftstoffen und Schmiermitteln auftreten kann, wie beispielsweise Stoßfänger ggf. in Form geeigneter Blends mit ABS oder geeigneten Kautschuken.
22. Für Sportartikel, wie z.B. Slalomstangen oder Skischuhschnallen.
23. Für Haushaltsartikel, wie z.B. Küchenspülen und Briefkastengehäuse.
24. Für Gehäuse, wie z.B. Elektroverteilerschränke.
25. Gehäuse für Elektrozahnbürsten und Föngehäuse
26. Transparente Waschmaschinen - Bullaugen mit verbesserter Beständigkeit gegenüber der Waschlösung.
27. Schutzbrillen, optische Korrekturbrillen.
28. Lampenabdeckungen für Kücheneinrichtungen mit verbesserter Beständigkeit gegenüber Küchendunst insbesondere Öldämpfen.
29. Verpackungsfolien für Arzneimittel.
30. Chip-Boxen und Chip-Träger
31. Für sonstige Anwendungen, wie z.B. Stallmasttüren oder Tierkäfige.

Die Formkörper und Extrudate aus den erfindungsgemäßen Polymeren sind ebenfalls Gegenstand dieser Anmeldung.

### Beispiele

### Beispiel 1

### Darstellung von 4-Hydroxybenzoesäurecyclooctylester

In einem Rundkolben mit Destillationsbrücke werden unter Argon 22.82 g (0.15 mol) p-Hydroxybenzoesäuremethylester, 38.47 g (0.30 mol) Cyclooctanol und 26 mg (0.1 mmol) Tetraisopropylorthotitanat vorgelegt. Das Gemisch wird gerührt und innerhalb 45 Minuten auf 180°C erwärmt. Der Druck wird auf 800 mbar und im weiteren Verlauf der Destillation auf 650 mbar reduziert. Nach weiteren 15 Minuten wird der Druck auf 900 mbar erhöht und das Reaktionsgemisch weitere 2 Stunden gerührt, wobei weiterhin Methanol abdestilliert wird. Es werden nochmals 9.62 g (0.075 mol) Cyclooctanol und 2 Tropfen Tetraisopropylorthotitanat zur Reaktionslösung gegeben und weitere 4 Stunden bei 180°C gerührt. Man lässt abkühlen und löst den Rückstand in 100 ml Ethylacetat und wäscht mehrmals mit dest. Wasser. Die organische Phase wird eingeengt. Das Rohprodukt wird über Säulenchromatographie (Kieselgel 60, 0040-0.063 mm, Merck) mit einem n-Hexan/Aceton Gemisch (3:1) gereinigt. Das erhaltene Produkt wird im Hochvakuum von anhaftendem Cyclooctanol befreit. Der weiße kristalline Rückstand wird in Aceton umkristallisiert Man erhält 4.70 g (12 %) farblose Kristalle.
Smp.: 109-111°C
¹H-NMR (400 MHz, CDCl₃) δ = 7.94 (d, 2 H), 6.87 (d, 2 H), 6.15 (s, 1 H), 5.21-5.13 (m, 1H), 2.00-1.72 (m, 6 H), 1.70-1.45 (m, 8 H).

### Beispiel 2

### Darstellung von 4-Hydroxybenzoesäurecyclododecylester

In einem Rundkolben mit Destillationsbrücke werden unter Argon 22.82 g (0.15 mol) p-Hydroxybenzoesäuremethylester, 55.30 g (0.30 mol) Cyclododecanol und 28 mg (0.1 mmol) Tetraisopropylorthotitanat vorgelegt. Das Gemisch wird gerührt und innerhalb von einer Stunde auf 160°C erwärmt. Bei Erreichen dieser Temperatur wird der Druck auf 900 mbar und im weiteren Verlauf der Reaktion auf 800 mbar reduziert wobei Methanol abdestilliert. Man lässt 5 Stunden bei dieser Temperatur rühren. Es werden nochmals 0.05 ml Tetraisopropylorthotitanat zugegeben und das Reaktionsgemisch weitere 20 Stunden bei 170°C bei Normaldruck gerührt. Der Rückstand wird einer Säulenchromatographie (Kieselgel 60, 0040-0.063 mm, Merck) unterworfen. Als Eluent dient ein n-Hexan/Aceton Gemisch (5:1). Das auf diese Weise erhaltene Produkt wird im Hochvakuum bei 140°C von anhaftendem Cyclododecanol befreit . Der Rückstand wird in Aceton umkristallisiert. Man erhält 5.9 g (13 %) in Form farbloser Kristalle:
¹H-NMR (400 MHz, CDCl₃) δ = 7.94 (d, 2H), 6.86 (d, 2 H), 6.08 (s, 1 H), 5.27-5.19 (m, 1 H), 1.89-1.75 (m,2 H), 1.68-1.55 (m, 2 H), 1.53-1.25 (m, 18 H).

### Beispiel 3

### Darstellung von 3-Hydroxybenzoesäurecyclooctylester

In einem Rundkolben mit Destillationsbrücke werden unter Argon 15.22 g (0.10 mol) m-Hydroxybenzoesäuremethylester, 25.64 g (0.20 mol) Cyclooctanol und 15 mg (0.05 mmol) Tetraisopropylorthotitanat vorgelegt. Das Gemisch wird gerührt und auf 170-180°C erwärmt. Man lässt 3.5 Stunden rühren und gibt nochmals 12.82 g (0.10mol) Cyclooctanol hinzu. Nach weiteren 5 Stunden bei 170-180°C werden nochmals 12.8 g Cyclooctanol und 15 mg Tetraisopropylorthotitanat zur Lösung hinzugegeben und weitere 7 Stunden bei Temperaturen zwischen 190 und 210°C gerührt.

Man lässt abkühlen und gibt ca. 500 ml dest. Wasser zur Reaktionslösung. Es wird mehrfach mit Diethylether extrahiert. Die vereinigten organischen Phasen werden mit gesättigter NaCl-Lösung gewaschen. Nach Trocknung über Magnesiumsulfat wird das Lösungsmittel im Vakuum entfernt. Das Rohprodukt wird mit einem Gemisch aus n-Hexan und Ethylacetat (5:1) an Kieselgel (Kieselgel 60, 0040-0.063 mm, Merck) chromatographiert. Um restliches Cyclooctanol zu entfernen wird das Produkt unter Hockvakuum erwärmt. Man erhält 16.03 g (65 %) in Form eines braunen viskosen Öls.
¹H-NMR (400 MHz, CDCl₃) δ = 7.68-7.65 (m, 1 H), 7.61-7.55 (m, 1 H), 7.32-7.23 (m, 1 H), 7.12-7.05 (m, 1 H), 6.96 (s, 1 H), 5.24-5.12 (m, 1 H), 1.77-1.45 (m, 14 H).

### Beispiel 4

### Darstellung von 3-Hydroxybenzoesäurecyclododecylester

In einem Rundkolben mit Destillationsbrücke werden unter Argon 15.22 g (0.10 mol) m-Hydroxybenzoesäuremethylester, 38.86 g (0.20 mol) Cyclododecanol und 19 mg (0.07 mmol) Tetraisopropylorthotitanat vorgelegt. Das Reaktionsgemisch wurde auf 175°C erwärmt und der Druck auf 800 mbar reduziert. Innerhalb von 2 Stunden wurde der Druck kontinuierlich auf 500 mbar gesenkt. Nach weiteren 4 Stunden werden noch 3 Tropfen Tetraisopropylorthotitanat zugesetzt. Der Druck wird auf 800 mbar erhöht. Es wurde noch 24 Stunden bei 170-200°C gerührt. Man lässt abkühlen und reinigt den Rückstand über Säulenchromatographie an Kieselgel (Kieselgel 60, 0040-0.063 mm, Merck) mit einem n-Hexan/Aceton Gemisch (5:1) gereinigt. Das erhaltene Produkt wird im Hochvakuum von anhaftendem Cyclododecanol befreit und nochmals an Kieselgel (Kieselgel 60, 0040-0.063 mm), Merck mit Dichlormethan chromatographiert. Man erhält 5.2 g (17 %) eines braun-gelben Feststoffs.
¹H-NMR (400 MHz, CDCl₃) δ = 7.61-7.52 (m, 2 H), 7.35-7.22 (m, 1 H), 7.08-7.00 (m, 1 H), 5.56 (s, 1 H), 5.32-5.28 (m, 1 H), 1.90-1.75 (m, 2 H), 1.74-1.56 (m, 2 H), 1.54-1.22 (m, 18 H).

### Beispiel 5

### Darstellung von Polycarbonat mit erfindungsgemäßer Endgruppe

In einem Kolben werden bei Raumtemperatur 17.12 g (0.075 mol) 2,2-Bis-(4-hydroxyphenyl)-propan und 6.60 g NaOH (220 mol-%, bezogen auf Bisphenol-Komponente) in 273 ml Wasser unter Stickstoffatmosphäre gelöst. Zu diesem Gemisch werden 1.12 g (6 mol-% bezogen auf Bisphenol-Komponente) 3-Hydroxybenzoesäurecyclooctylester (s. Beispiel 3) gelöst in 273 ml Dichlormethan gegeben. Man lässt für 5 Minuten Rühren. Bei Raumtemperatur (25°C) und unter kräftigem Rühren werden 14.73 g (200 mol-%, bezogen auf Bisphenol-Komponente) Phosgen eingeleitet. Der pH-Wert wird dabei durch Nachdosieren von 40%iger NaOH-Lösung im Bereich von pH = 12.5-13.5 gehalten. Nach beendeter Einleitung wird die Apparatur 5 Minuten mit Stickstoff gespült. Man gibt zum Reaktionsgemisch 0.085 g (1 mol-%) *N-*Ethylpiperidin gelöst in 10 ml Dichlormethan. Man lässt noch 45 Minuten Rühren. Daraufhin wird mit Dichlormethan verdünnt und die organische Phase abgetrennt. Nach Extraktion der organischen Phase mit gleichem Volumen an 10 %iger Salzsäure wird die organische Phase abgetrennt und noch 5 x mit Wasser elektrolytfrei gewaschen. Das in der organischen Phase gelöste Polymer wird in Methanol gefällt und im Vakuum getrocknet.
Ausbeute: 18.90 g (vor Fällung)
Mₙ = 10005 g/mol
M_{w} = 19163 g/mol
D = 1.92
T_{g} = 147°C

### Beispiel 6

### Darstellung von Polycarbonat mit erfindungsgemäßer Endgruppe

Die Ausführung des Versuchs entspricht der Vorschrift von Beispiel 5. Abweichend wird als Kettenabbrecher-statt 3-Hydroxybenzoesäurecyclooctylester 6 mol-% (bezogen auf Bisphenol-Komponente) 3-Hydroxybenzoesäurecyclododecylester (s. Bsp. 4) eingesetzt.
Mₙ = 8384
M_{w} = 20423
D = 2.44
T_{g} =142°C

### Beispiel 7

### Vergleichsbeispiel

Die Ausführung des Versuchs entspricht der Vorschrift von Beispiel 5. Abweichend wird der Kettenabbrecher schon vor der Phosgenierung im Zweiphasensystem gelöst. Als Kettenabbrecher wird statt 3-Hydroxybenzoesäurecyclooctylester 6 mol-% (bezogen auf Bisphenol-Komponente) 4-tert.-Butylphenol eingesetzt.
Mₙ = 10238
M_{w} = 19431
D = 1.90
T_{g} =148°C
Beispiel 8

### Vergleichsbeispiel

Die Ausführung des Versuchs entspricht der Vorschrift von Beispiel 5. Abweichend wird als Kettenabbrecher statt 3-Hydroxybenzoesäurecyclooctylester 5 mol-% (bezogen auf Bisphenol-Komponente) 4-Hydroxybenzoesäurecyclooctylester eingesetzt.
Mₙ = 11679
M_{w} = 22793
D = 1.95
T_{g} = 149°C

### Beispiel 9:

### Darstellung von 2-Cyclododecylphenol

83 g (0,499 mol) Cyclododecen (Aldrich), 470,7 g (5,00 mol) frisch destilliertes Phenol und 276 g des Ionenaustauschers Lewatit S100, sauer (Bayer AG) werden in einem Rundkolben vorgelegt. Das Reakrtionsgemisch wir auf 73 - 74°C erhitzt. Es wird für 24 Stunden bei 74°C gerührt. Nach Abkühlen auf Raumtemperatur wird vom Ionenaustauscher abfiltriert. Das Reaktionsgemisch wird unter reduziertem Druck destilliert um Phenol zu entfernen. Der Rückstand wird an Kieselgel mit Toluol chromatographiert. Das so erhaltene Produkt wird im Vakuum getrocknet um überschüssiges Toluol zu entfernen. Man erhält 91,2 g (70 %) eines weißen Feststoffs.
¹H-NMR (400 MHz, CDCl₃) δ = 9.10 (s, 1 H), 7.1-7.0 (m, 1 H), 7.0-6.85 (m, 1 H), 6.85-6.65 (m, 2 H), 3.30 - 3.15 (m, 1 H), 1.85 - 1.05 (m, 22 H).

### Beispiel 10

### Darstellung von Polycarbonat mit erfindungsgemäßer Endgruppe

In einem Kolben werden bei Raumtemperatur 22.83 g (0.1 mol) 2,2-Bis-(4-hydroxyphenyl)-propan und 8.80 g NaOH (220 mol-%, bezogen auf Bisphenol-Komponente) in 273 ml Wasser unter Stickstoffatmosphäre gelöst. Zu diesem Gemisch werden 2.865 g (11 mol-% bezogen auf Bisphenol-Komponente) 2-Cyclododecylphenol (s. Beispiel 9) gelöst in 173 ml Dichlormethan gegeben. Man lässt für 5 Minuten Rühren. Bei Raumtemperatur (25°C) und unter kräftigem Rühren werden 20.62 g (200 mol%, bezogen auf Bisphenol-Komponente) Phosgen eingeleitet. Der pH-Wert wird dabei durch Nachdosieren von 40%iger NaOH-Lösung im Bereich von pH = 12.5-13.5 gehalten. Nach beendeter Einleitung wird die Apparatur 5 Minuten mit Stickstoff gespült. Man gibt zum Reaktionsgemisch 0.11 g (1 mol-%) *N*-Ethylpiperidin gelöst in 10 ml Dichlormethan. Man lässt noch 45 Minuten Rühren. Daraufhin wird mit Dichlormethan verdünnt und die organische Phase abgetrennt. Nach Extraktion der organischen Phase mit gleichem Volumen an 10 %iger Salzsäure wird die organische Phase abgetrennt und noch 5 × mit Wasser elektrolytfrei gewaschen. Das in der organischen Phase gelöste Polymer wird in Methanol gefällt und im Vakuum getrocknet.
Ausbeute: 23.67 g (vor Fällung)
Mₙ = 10205 g/mol
M_{w} = 18635 g/mol
D = 1.83
T_{g} = 133°C

### Erfindungsgemäße Endgruppen:

| Ex. no. | Terminal group used | Zero shear-rate viscösity (Pa.s) (at 270°C) | Molecular weight (g/mol) | Glass transition temperature (°C) |
|---|---|---|---|---|
| 5 | 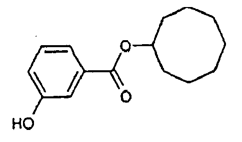 | 140 | M_{w} = 19163 | 147 |
| | | | Mₙ = 10005 | |
| 6 | 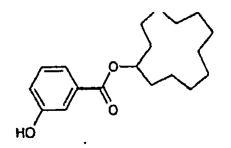 | 75 | M_{w} = 20423 | 142 |
| | | | Mₙ = 8384 | |
| 10 | 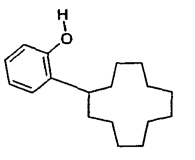 | 240 | M_{w} = 18635 | 133 |
| | | | Mₙ = 10205 | |

### Vergleichsbeispiele:

| Bsp.-Nr.: | Eingesetzte Endgruppe | Nullviskosität (Pa.s) (bei 270°C) | Molgewicht (g/mol) | Glasübergangstemperatur (°C) |
|---|---|---|---|---|
| | 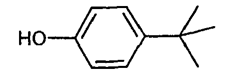 | 350 | M_{w} = 19431 | 148 |
| 7 | | | Mₙ = 10238 | |
| | 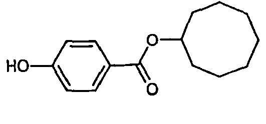 | 510 | M_{w} = 22793 | 149 |
| 8 | | | Mₙ =11679 | |

Anhand vorstehend gezeigter Tabellen wird ersichtlich, dass die erfindungsgemäßen. Polycarbonate im Vergleich zu Polycarbonaten mit mit p-substituierten Phenolen, wie z.B. mit herkömmlichen Endgruppen wie *tert*.-Butylphenol, bei annähernd identischem Molekulargewicht überraschenderweise eine reduzierte Nullviskosität aufweisen.

## Patentansprüche

1. Polycarbonate, Polyestercarbonate und Polyester, wobei die Endgruppen aus Kettenabbrechern gemäß Verbindungen der Formel (1) resultieren, 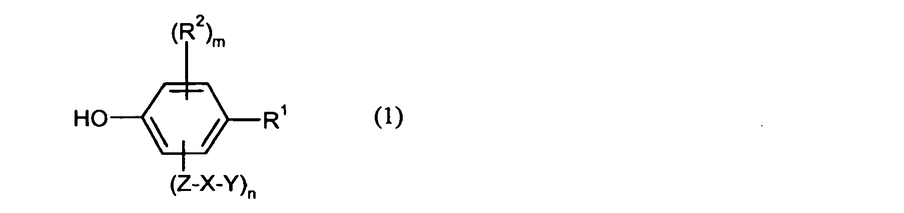 wobei
R¹ entweder H oder ein CH₃-Rest ist;
R² für H, lineares oder verzweigtes C₁-C₁₈ Alkyl- oder Alkoxy-, Cl oder Br oder für einen gegebenenfalls substituierten Aryl- oder Aralkylrest steht,
Z für einen Alkylspacer mit 1 bis 30 Kohlenstoffatomen oder für eine Einfachbindung steht,
X für ein divalentes Radikal wie -O-, -CO-, -CH₂-, -COO- oder -OCO₂- steht,
Y für einen gegebenenfalls substituierten cycloaliphatischen Rest oder ein gegebenenfalls substituierten polycyclischen aliphatischen Rest wie Adamantyl- oder Norbornylrest oder ein gegebenenfalls substituierter aromatischer Rest steht,
und
n die Werte 1, 2, 3, 4 und m die Werte 0, 1, 2, 3 annehmen kann, wobei n und m in Summe 4 ergeben müssen
oder
X für eine Einfachbindung steht und es sich im Falle, dass X für eine Einfachbindung steht bei den Verbindungen der Formel (1) um o-Cyclododecylphenol, o-Cyclooctylphenol, m-Cyclododecylphenol oder m-Cyclooctylphenol handelt.

2. Polycarbonate, Polyestercarbonate und Polyester gemäß Anspruch 1, **dadurch gekennzeichnet, dass** die resultierenden Endgruppen solche der Formeln (5a) bis (5f) sind, 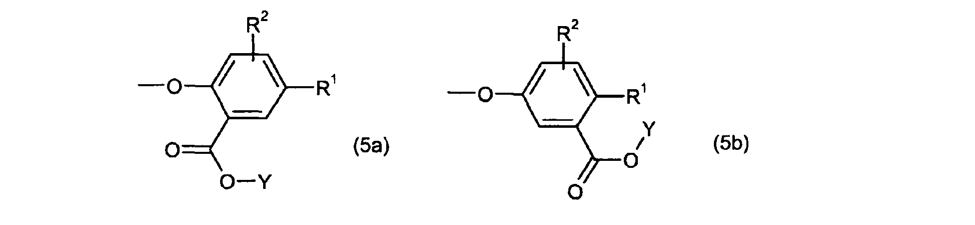 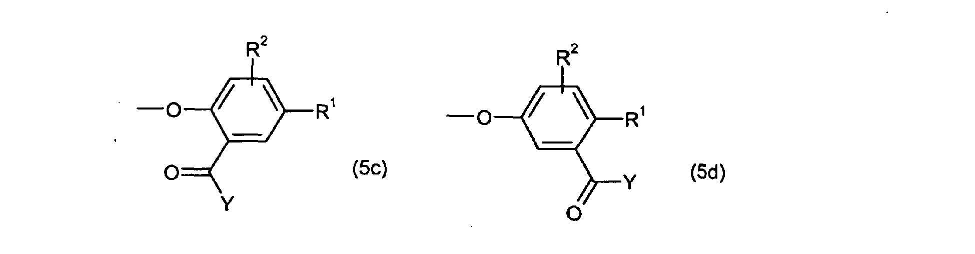 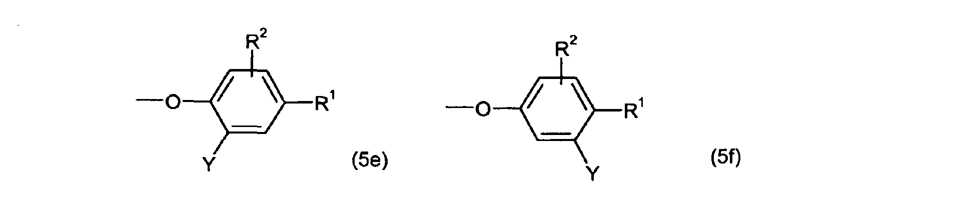 worin die Reste Y, R¹ und R² die in Anspruch 1 genannten Bedeutungen haben und es sich im Falle der Formeln (5e) und (5f) um die aus o-Cyclododecylphenol, o-Cyclooctylphenol, m-Cyclododecylphenol oder m-Cyclooctylphenol resultierenden Endgruppen handelt.

3. Polycarbonate, Polyestercarbonate und Polyester gemäß Anspruch 1, **dadurch gekennzeichnet, dass** die Endgruppen aus den Verbindungen o-Cyclododecylphenol, o-Cyclooctylphenol, m-Cyclododecylphenol, m-Cyclooctylphenol, 3-Hydroxybenzophenon, 2-Hydroxybenzophenon, 3-Hydroxybenzoesäurephenylester, 2-Hydroxybenzoesäurephenylester, 3-Hydroxybenzoesäure-(4-tert.-butylphenyl)ester, 2-Hydroxybenzoesäure-(4-tert.-butylphenyl)ester, 3-Hydroxybenzoesäure-(4-methylphenyl)ester, 2-Hydroxybenzoesäure-(4-methylphenyl)ester, 3-Hydroxybenzoesäurecyclohexylester, 2-Hydroxybenzoesäurecyclohexylester 3-Hydroxybenzoesäurecyclooctylester, 2-Hydroxybenzoesäurecyclooctylester 3-Hydroxybenzoesäurecyclododecylester oder 2-Hydroxybenzoesäurecyclododecylester resultieren.

4. Verwendung der Polycarbonate, Polyestercarbonate und Polyester gemäß Anspruch 1 zur Herstellung von Formkörpern und Extrudaten.

5. Extrudate und Formkörper aus Polycarbonaten gemäß Anspruch 1.

6. Verfahren zur Herstellung der Polycarbonate Polyestercarbonate und Polyester gemäß Anspruch 1 nach prinzipiell bekannten Verfahren, **dadurch gekennzeichnet, dass** 0,5 bis 8 mol %, bezogen auf die Gesamtmole an Diphenolen, der Verbindungen der Formel (1) gemäß Anspruch 1 eingesetzt werden.

7. Verfahren gemäß Anspruch 6, **dadurch gekennzeichnet, dass** 2 bis 6 mol-%, bezogen auf die Gesamtmole an Diphenolen, der Verbindungen der Formel (1) gemäß Anspruch 1 eingesetzt werden.

## Claims

1. Polycarbonates, polyester carbonates and polyesters, wherein the terminal groups result from chain terminators according to compounds having formula (1) 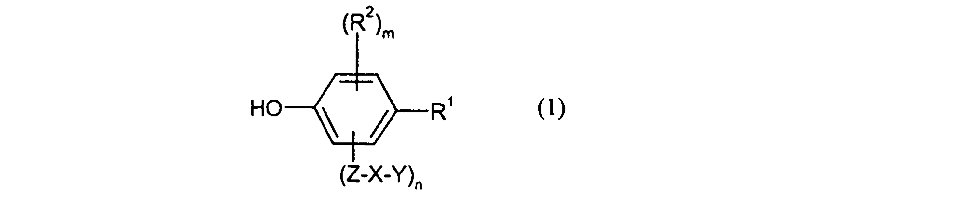 wherein
R¹ is either H or a CH₃ radical;
R² stands for H, linear or branched C₁-C₁₈ alkyl or alkoxy, Cl or Br or for an optionally substituted aryl or aralkyl radical,
Z stands for an alkyl spacer having 1 to 30 carbon atoms or for a single bond,
X stands for a divalent radical such as -O-,-CO-, -CH₂-, -COO- or -OCO₂-,
Y stands for an optionally substituted cycloaliphatic radical or an optionally substituted polycyclic aliphatic radical such as adamantyl or norbornyl radical or an optionally substituted aromatic radical,
and
n can assume the values 1, 2, 3, 4 and m the values 0, 1, 2, 3, wherein the sum of n plus m must be 4
or
X stands for a single bond, and where X stands for a single bond the compounds having formula (1) are o-cyclododecylphenol, o-cyclooctylphenol, m-cyclododecylphenol or m-cyclooctylphenol.

2. Polycarbonates, polyester carbonates and polyesters according to claim 1, **characterised in that** the resulting terminal groups are those having formulae (5a) to (5f), 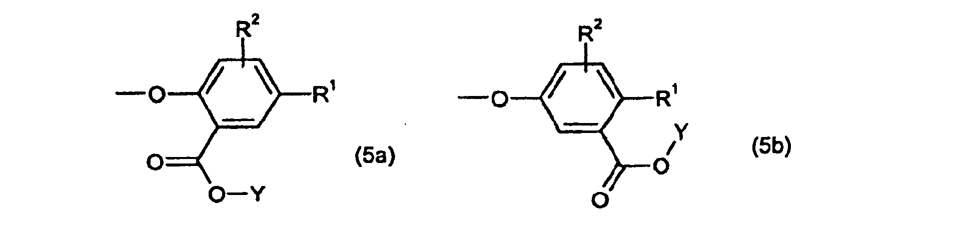 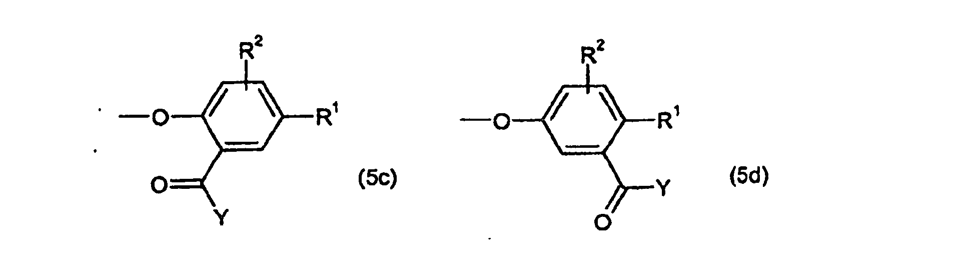 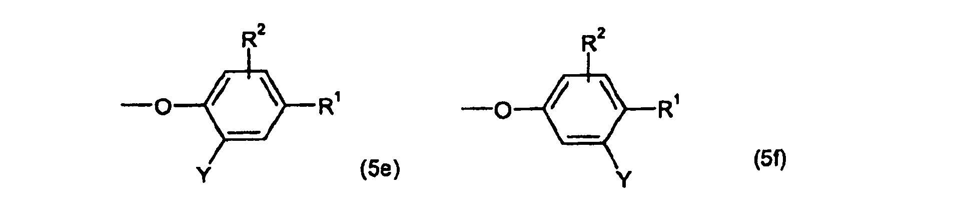 wherein the radicals Y, R¹ and R² have the meanings given in claim 1 and formulae (5e) and (5f) are the terminal groups resulting from o-cyclododecylphenol, o-cyclooctylphenol, m-cyclododecylphenol or m-cyclooctylphenol.

3. Polycarbonates, polyester carbonates and polyesters according to claim 1 **characterised in that** the terminal groups result from the compounds o-cyclododecylphenol, o-cyclooctylphenol, m-cyclododecylphenol, m-cyclooctylphenol, 3-hydroxybenzophenone, 2-hydroxybenzophenone, 3-hydroxybenzoic acid phenyl ester, 2-hydroxybenzoic acid phenyl ester, 3-hydroxybenzoic acid (4-tert-butylphenyl) ester, 2-hydroxybenzoic acid (4-tert-butylphenyl) ester, 3-hydroxybenzoic acid (4-methylphenyl) ester, 2-hydroxybenzoic acid (4-methylphenyl) ester, 3-hydroxybenzoic acid cyclohexyl ester, 2-hydroxybenzoic acid cyclohexyl ester, 3-hydroxybenzoic acid cyclooctyl ester, 2-hydroxybenzoic acid cyclooctyl ester, 3-hydoxybenzoic acid cyclododecyl ester or 2-hydroxybenzoic acid cyclododecyl ester.

4. Use of the polycarbonates, polyester carbonates and polyesters according to claim 1 for the production of moulded parts and extrudates.

5. Extrudates and moulded parts made from polycarbonates according to claim 1.

6. Process for the production of the polycarbonates, polyester carbonates and polyesters according to claim 1 by processes known in principle, **characterised in that** 0.5 to 8 mol%, based on the total moles of diphenols, of compounds having formula (1) according to claim 1 are used.

7. Process according to claim 6, **characterised in that** 2 to 6 mol%, based on the total moles of diphenols, of compounds having formula (1) according to claim 1 are used.

## Revendications

1. Polycarbonates, carbonates de polyester et polyesters, les groupes terminaux résultant de casseurs de chaîne selon les composés de la formule (1), 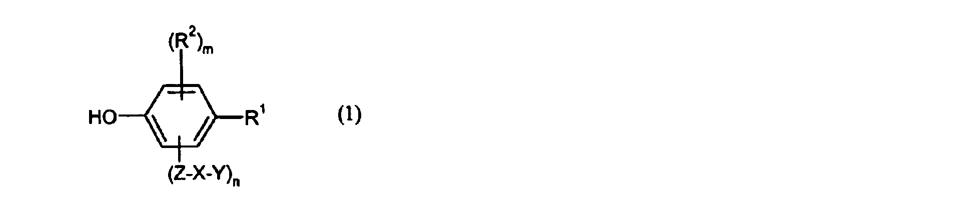 où
R¹ est soit H soit un reste CH₃;
R² représente H, un groupe alkyle ou alcoxy en C₁-C₁₈ linéaire ou ramifié, Cl ou Br ou un reste aryle ou aralkyle le cas échéant substitué,
Z représente un espaceur alkyle avec 1 à 30 atomes de carbone ou une liaison simple,
X représente un radical bivalent tel que -O-, -CO-, -CH₂-, -COO- ou -OCO₂-,
Y représente un reste cycloaliphatique le cas échéant substitué ou un reste aliphatique polycyclique le cas échéant substitué tel qu'un reste adamantyle ou norbornyle, ou un reste aromatique le cas échéant substitué,
et
n peut prendre les valeurs 1, 2, 3, 4 et m les valeurs 0, 1, 2, 3, la somme de n et m devant être 4
ou
X représente une liaison simple et, dans le cas où X représente une liaison simple pour les composés de la formule (1), il s'agit de o-cyclododécylphénol, o-cyclooctylphénol, m-cyclododécylphénol ou m-cyclooctylphénol.

2. Polycarbonates, carbonates de polyester et polyesters suivant la revendication 1, **caractérisés en ce que** les groupes terminaux résultant sont ceux des formules (5a) à (5f), 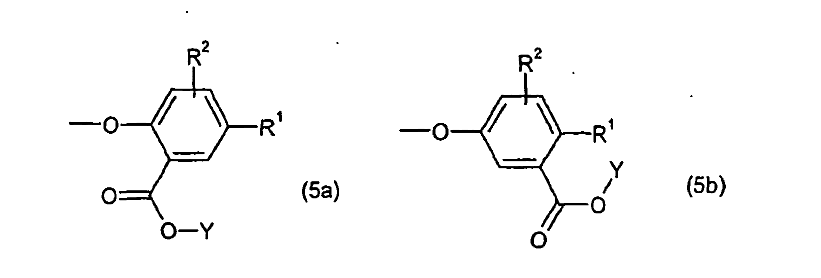 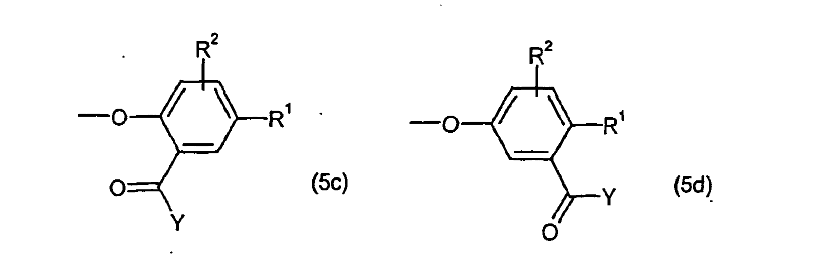 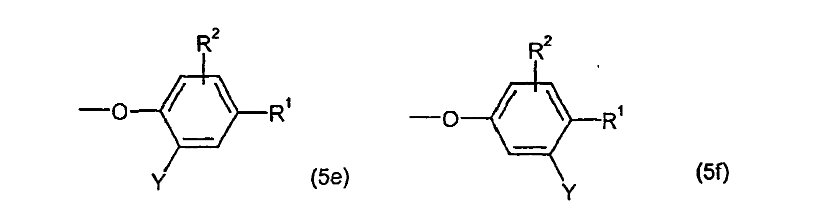 où les restes Y, R¹ et R² ont les significations données dans la revendication 1 et, dans le cas des formules (5e) et (5f), il s'agit des groupes terminaux résultant de l'o-cyclododécylphénol, de l'o-cyclooctylphénol, du m-cyclododécylphénol ou du m-cyclooctylphénol.

3. Polycarbonates, carbonates de polyester et polyesters suivant la revendication 1, **caractérisés en ce que** les groupes terminaux résultent des composés o-cyclododécylphénol, o-cyclooctylphénol, m-cyclododecylphénol, m-cyclooctylphénol, 3-hydroxybenzophénone, 2-hydroxybenzophénone, 3-hydroxybenzoate de phényle, 2-hydroxybenzoate de phényle, 3-hydroxybenzoate de 4-tert.-butylphényle, 2-hydroxybenzoate de 4-tert.-butylphényle, 3-hydroxybenzoate de 4-méthylphényle, 2-hydroxybenzoate de 4-méthylphényle, 3-hydroxybenzoate de cyclohexyle, 2-hydroxybenzoate de cyclohexyle, 3-hydroxybenzoate de cyclooctyle, 2-hydroxybenzoate de cyclooctyle, 3-hydroxybenzoate de cyclododécyle ou 2-hydroxybenzoate de cyclododécyle.

4. Utilisation des polycarbonates, carbonates de polyester et polyesters suivant la revendication 1 pour la fabrication de corps moulés et de produits d'extrusion.

5. Produits d'extrusion et corps moulés fabriqués à partir des polycarbonates suivant la revendication 1.

6. Procédé de fabrication des polycarbonates, carbonates de polyester et polyesters suivant la revendication 1 selon des procédés en principe connus, **caractérisé en ce qu'**on utilise 0,5 à 8 % en mole, rapportés aux moles totales de diphénols, des composés de la formule (1) suivant la revendication 1.

7. Procédé de fabrication suivant la revendication 6, **caractérisé en ce qu'**on utilise 2 à 6 % en mole, rapportés aux moles totales de diphénols, des composés de la formule (1) suivant la revendication 1.
